# EUROPÄISCHE PATENTSCHRIFT

(11) **EP 0 384 365 B1**
(45) Veröffentlichungstag und Bekanntmachung des Hinweises auf die Patenterteilung: **08.12.1993**
(21) Anmeldenummer: 90103175.7
(22) Anmeldetag: 20.02.1990
(51) Int. Cl.: C07D 249/08, A01N 43/653

(54) **0-(1,2,4-Triazolyl-1)-0-Phenyl-Acetale und diese enthaltende Fungizide**
0-(1,2,4-Triazolyl-1)-0-phenyl acetals and fungicides containing them
0-(1,2,4-triazolyl-1)-0-phénylacétals et fongicides les contenant

(30) Priorität: 24.02.1989 DE 3905766
(43) Veröffentlichungstag der Anmeldung: 29.08.1990
(73) Patentinhaber: BASF Aktiengesellschaft, 67063 Ludwigshafen (DE)
(72) Erfinder: Baus, Ulf, Dr., D-6915 Dossenheim (DE); Reuther, Wolfgang, Dr., D-6900 Heidelberg (DE); Lorenz, Gisela, Dr., D-6730 Neustadt (DE); Ammermann, Eberhard, Dr., D-6700 Ludwigshafen (DE)

(56) Entgegenhaltungen:
- EP-A- 0 002 671
- EP-A- 0 121 171
- US-A- 4 147 791

## Beschreibung

Die vorliegende Erfindung betrifft neue N-Hydroxitriazolderivate, ihre Salze und Metallkomplexe, ein Verfahren zu ihrer Herstellung und ihre Verwendung als Fungizide.

Es ist bekannt, Triazolyl-O,N-acetale, z.B. 1-(4-Chlorphenoxi)-3,3-dimethyl-1-(1,2,4-triazol-1-yl)-butan-2-on (DE 22 01 063, US-4 147 791) oder 1-(4-Chlorphenoxi)-3,3-dimethyl-1-(1,2,4-triazol-1-yl)-butan-2-ol als Fungizide (DE 23 24 010) zu verwenden. Es ist ferner bekannt, Triazolylglykoläther als Fungizide zu verwenden (EP-A-2671). Ihre Wirkung ist jedoch nicht immer befriedigend.

Es wurde nun gefunden, daß Verbindungen der allgemeinen Formel I 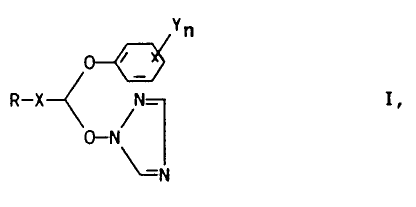
in der
- R: einen tert.-Alkylrest mit 4 bis 6 C-Atomen oder einen Phenylrest, der gegebenenfalls durch Halogen, Phenyl, Phenyloxi, C₁-C₄-Alkoxi substituiert ist,
- X: C = 0 oder CHOH
- Y: Wasserstoff, Alkyl mit 1 bis 9 C-Atomen, C₁-C₄-Alkyloxi, Halogen, Phenyl, Phenoxi und
- n: 1 bis 5
bedeuten, und ihre Salze und Metallkomplexverbindungen eine überraschend gute fungizide Wirkung haben.
- R: bedeutet beispielsweise tert.-Butyl
oder Phenyl, das gegebenenfalls ein- bis fünffach (bis dreifach) durch Halogen (Cl, Br, F), Phenoxi, C₁-C₄-Alkoxi (Methoxi, Ethoxi, tert.-Butoxi) substituiert ist.
- X: bedeutet C=O oder CHOH,
- Y: bedeutet beispielsweise C₁-C₄-Alkyl (Methyl, Ethyl, tert.-Butyl), C₁-C₄-Alkoxi (Methoxy, Ethoxi, tert.-Butoxi), Halogen (Cl, Br, F), Phenyl, Phenoxi, z.B. 4-Chlor oder 3-Methyl-4-chlor.
- n: bedeutet beispielsweise 1, 2, 3, 4 oder 5, wobei für n größer als 1 Yₙ gleich oder verschieden ist.

Salze sind beispielsweise die pflanzenverträglichen Säureadditionssalze, z.B. die Salze mit anorganischen oder organischen Säuren, wie beispielsweise die Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions beliebig ist.

Ferner lassen sich die Verbindungen der Formel I nach bekannten Methoden in Metallkomplexe überführen. Das kann durch Umsetzung dieser Verbindungen mit Metallsalzen, z.B. der Metalle Kupfer, Zink, Eisen, Mangan oder Nickel, beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Es wurde ferner gefunden, daß sich die Verbindungen der allgemeinen Formel I (X = C=0) sehr leicht und in guten Ausbeuten durch Umsetzung von 1-Hydroxi-1,2,4-triazol mit den Verbindungen der allgemeinen Formel II, 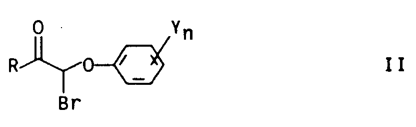
in der
R, Y und n die oben angegebenen Bedeutungen haben, herstellen lassen. Die Verbindungen der allgemeinen Formel II sind bekannt oder lassen sich durch bekannte Verfahren herstellen (z.B. DE 22 01 063). 1-Hydroxi-1,2,4-triazol wird z.B. wie folgt hergestellt.

103,5 g (1,5 mol) 1-H-1,2,4-Triazol wurden in 1344 g (12 mol) 50 %igem wäßrigem Kaliumhydroxid gelöst. Unter Eiskühlung wurden 340 g (3 mol) 30 %iges H₂O₂ und portionsweise 555 g (3,75 mol) Phthalsäureanhydrid zugegeben und 2 Stunden bei Raumtemperatur (20 bis 30°C) gerührt. Anschließend wurde mit ca. 35 %iger Schwefelsäure auf einen pH-Wert unter 1,5 angesäuert, der entstandene Niederschlag abgesaugt und das Filtrat wie üblich aufgearbeitet. Man erhielt 19 g 1-Hydroxy-1,2,4-Triazol, Fp. 132°C. Das ist eine Ausbeute von 15 % der Theorie.

Die Umsetzung mit der Verbindung II erfolgt beispielsweise in einem inerten organischen Lösemittel wie Tetrahydrofuran (THF), Dimethylsulfoxid (DMSO), Dimethylformamid (DMF), Diethylether, vorzugsweise THF oder THF/Wasser-Gemisch in Gegenwart einer Base wie Triethylamin, Tributylamin, NaOH, Natriumcarbonat oder Pyridin bei Temperaturen zwischen 0 und 100°C, vorzugsweise bei Raumtemperatur (20°C). Die Verbindungen der allgemeinen Formel I (X = C=O) entstehen als Racemate, die in üblicher Weise in ihre Isomeren getrennt werden können. Sowohl die reinen Enantiomeren als auch ihre Gemische sind Gegenstand der Erfindung und als Fungizide geeignet.

Es wurde weiterhin gefunden, daß sich die Verbindungen der allgemeinen Formel I (X = C=O) mit üblichen Reduktionsmitteln wie NaBH₄, Lithiumaluminiumhydrid (LAH) oder NaCNBH₃ in die Verbindungen der allgemeinen Formel I (X = CHOH) überführen lassen. Die Verbindungen der allgemeinen Formel I (X = CHOH) entstehen als racemische Diastereomerengemische, die in üblicher Weise in ihre Isomeren getrennt werden können. Sowohl die reinen Isomeren als auch ihre Gemische sind Gegenstand der Erfindung und als Fungizide geeignet.

### Herstellungsbeispiele

### Beispiel 1

1,7 g (20 mmol) 1-Hydroxi-1,2,4-triazol werden in 75 ml THF gelöst. Unter Rühren werden 6,1 g (20 mmol) 1-Brom-1-(4-chlor-phenoxi)-3,3-dimethylbutan-2-on, danach 2 g (20 mmol) Triethylamin zugegeben. Nach 2 Stunden wird der entstandene Niederschlag abgesaugt. Das Filtrat wird eingeengt, in Essigester/Ether (1:1) aufgenommen, mit Wasser gewaschen und getrocknet. Nach Entfernen des Lösemittels erhält man 6.2 g (100% der Theorie) eines öligen Rohproduktes der Verbindung Nr. 1, das aus Cyclohexan kristallisiert.
Fp.: 82°C

| | | | | |
|---|---|---|---|---|
| Analyse: C₁₄H₁₆ClN₃O₃ (309.61) | ber.: | C 54,3 | H 5,2 | N 13,5 |
| | gef.: | C 54,3 | H 5,4 | N 13,4 |

- HNMR (CDCl₃):: 1,27 (s, 9H); 6,35 (s, 1H); 6.97 - 7,37 (m, 4H); 7,78 (s, 1H); 8,1 (s, 1H) [ppm].

In entsprechender Weise wurden erhalten:

| Nr. | R | X | Y | phys. Daten | |
|---|---|---|---|---|---|
| 1 | C(CH₃)₃ | C=O | 4-Cl | Fp.: | 82°C |
| 3 | C(CH₃)₃ | C=O | 2,4-Cl₂ | Fp.: | 62-64°C |
| | | | | HNMR: | 1,28 (s, 9H); |
| | | | | | 6,32 (s, 1H); |
| | | | | | 7,08-7,43 (m, 3H); |
| | | | | | 7,78 (s, 1H); |
| | | | | | 8,18 (s, 1H). |

### Beispiel 2

### Reduktion der Verbindung Nr. 1 zum Alkohol Nr. 2:

4,64 g (15 mmol) 1-(4-Chlorphenoxi)-1-(1-oxi-1,2,4-triazolyl)-3,3-dimethyl-butan-2-on (Verbindung Nr. 1) werden in 100 ml THF/MeOH (1:1) gelöst. Danach werden unter Rühren 0,57 g (15 mmol) NaBH₄ zugegeben. Nach 1 Stunde wird in Wasser gegossen, schwach angesäuert und mit Essigester extrahiert. Die organische Phase wird mit Wasser gewaschen und getrocknet.

Nach Entfernen des Lösemittels erhält man 4,64 g (99% der Theorie) eines viskosen Öls (Verbindung Nr. 2).
- HNMR (CDCl₃):: 0,99 und 1,03 (2s, 9H); 3,33 und 3,75 (2d, 1H); 5.69 und 5.99 (2d, 2H); 6,8 (m, 4H); 7,74, 7,78, 7,82 und 8,02 (4s, 2H).

In entsprechender Weise wurden erhalten:

| Nr. | R | X | Y | phys. Daten | |
|---|---|---|---|---|---|
| 2 | C(CH₃)₃ | CHOH | 4-Cl | siehe Beispiel 2 | |
| 4 | C(CH₃)₃ | CHOH | 2,4-Cl₂ | HNMR (CDC₁₃): | 1,03 und 1.08 (2s, 9H); |
| | | | | | 3,36 und 3,47 (2d, 1H); |
| | | | | | 5,83 und 6,03 (2d, 1H); |
| | | | | | 6,7 - 7,45 (m, 3H); |
| | | | | | 7,73, 7,8, 7,82 und 8.08 (4s, 2H). |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:
I. Man vermischt 90 Gew.-Teile der Verbindung Nr. 1 (Tab. 1) mit 10 Gew.-Teilen N-Methyl-α-pyrrolidon und erhält eine Lösung, die zur Anwendung in Form kleinster Tropfen geeignet ist.
II. 20 Gew.-Teile der Verbindung Nr. 2 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.
III. 20 Gew.-Teile der Verbindung Nr. 4 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Nasser erhält man eine wäßrige Dispersion.
IV. 20 Gew.-Teile der Verbindung Nr. 1 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.
V. 80 Gew.-Teile der Verbindung Nr. 2 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.
VI. 3 Gew.-Teile der Verbindung Nr. 4 werden mit 97 Gew.-Teilen feinteiligem Kaolin innig vermischt. Man erhält auf diese Weise ein Stäubemittel, das 3 Gew.% des Wirkstoffs enthält.
VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 40 Gew.-Teile der Verbindung Nr. 2 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.
IX. 20 Gew.-Teile der Verbindung Nr. 4 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

### Anwendungsbeispiel 1

### Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20-22°C in eine Kammer mit hoher Luftfeuchtigkeit (90-95%) gestellt. Während dieser Zeit keimten die Sporen aus und die Keimschläuche drangen in das Blattgewebe ein. Die inoculierten Pflanzen wurden anschließend mit wäßrigen Spritzbrühen, die 80% Wirkstoff und 20% Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65-70% relativer Luftfeuchte aufgestellt. Nach 8 Tagen wurde das Ausmaß der Rostpilzentwicklung auf den Blättern ermittelt.

Das Ergebnis zeigt, daß die Wirkstoffe 1, 2 und 4 bei der Anwendung als 0,0125 %ige (Gew.%) Spritzbrühe eine gute fungizide Wirkung zeigen (100 %).

## Patentansprüche

1. Verbindungen der allgemeinen Formel I, 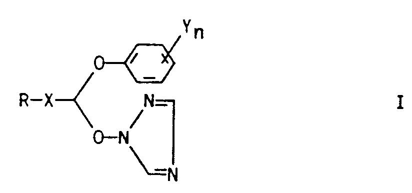 in der
R einen tert.-Alkylrest mit 4 bis 6 C-Atomen, einen Phenylrest, der gegebenenfalls durch Halogen, Phenyl, Phenoxi, C₁-C₄-Alkoxi substituiert ist,
X C = O oder CHOH ,
Y Wasserstoff, Alkyl mit 1 bis 9 C-Atomen, C₁-C₄-Alkyloxi, Halogen, Phenyl, Phenoxi und
n 1 bis 5
bedeuten, und ihre Salze und ihre Metallkomplexverbindungen.

2. Verfahren zur Herstellung von Verbindungen der allgemeinen Formel I, dadurch gekennzeichnet, daß man Verbindungen der allgemeinen Formel II, 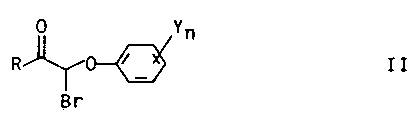 in der
R, Y und n die in Anspruch 1 angegebenen Bedeutungen haben, mit 1-Hydroxi-1,2,4-triazol umsetzt und die erhaltenen Verbindungen der allgemeinen Formel I (X = C=O) gegebenenfalls zu den Verbindungen der allgemeinen Formel I (X = CHOH) reduziert.

3. Fungizide Mittel, gekennzeichnet durch einen Gehalt an einem Trägerstoff und einer fungizid wirksamen Menge einer Verbindung der allgemeinen Formel I gemäß Anspruch 1.

4. Verfahren zur Bekämpfung von Pilzen, dadurch gekennzeichnet, daß man die Pilze oder die vom Pilzbefall bedrohten Pflanzen, Saatgüter, Materialien oder Flächen behandelt mit einer fungizid wirksamen Menge einer Verbindung der allgemeinen Formel I gemäß Anspruch 1.

5. Verbindungen der allgemeinen Formel I gemäß Anspruch 1, in der
R tertiär-Butyl,
X C=O und
Yₙ 4-Chlor oder 2,4-Dichlor
bedeuten.

6. Verbindungen der allgemeinen Formel I gemäß Anspruch 1, in der
R tertiär-Butyl,
X CHOH und
Yₙ 4-Chlor oder 2,4-Dichlor
bedeuten.

## Claims

1. A compound of the general formula I 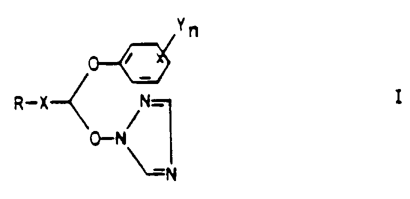 where
R is tert-alkyl of 4 to 6 carbons, or phenyl which may be substituted by halogen, phenyl, phenoxy or C₁-C₄-alkoxy,
X is C = O or CHOH,
Y is hydrogen, alkyl of 1 to 9 carbons, C₁-C₄-alkoxy, halogen, phenyl or phenoxy and
n is 1 to 5,
or a salt or metal complex thereof.

2. A process for preparing a compound of the general formula I, which comprises reacting a compound of the general formula II 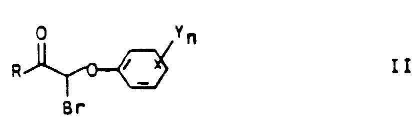 where
R, Y and n have the meanings specified in claim 1, with a 1-hydroxy-1,2,4-triazole and, if appropriate, reducing the resulting compound of the general formula I (X = C=O) to give a compound of the general formula I (X = CHOH).

3. A fungicide containing a carrier and a fungicidally effective amount of a compound of the general formula I as claimed in claim 1.

4. A method of controlling fungi, wherein the fungi, or the plants, seed, materials or areas threatened by fungal attack, are treated with a fungicidally effective amount of a compound of the general formula I as claimed in claim 1.

5. A compound of the general formula I as claimed in claim 1, where
R is tert-butyl,
X is C=O and
Yₙ is 4-chloro or 2,4-dichloro.

6. A compound of the general formula I as claimed in claim 1, where
R is tert-butyl,
X is CHOH and
Yₙ is 4-chloro or 2,4-dichloro.

## Revendications

1. Composés de formule générale I, 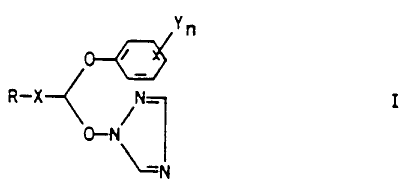 dans laquelle
R représente un reste ter-alkyle ayant 4 à 6 atomes C, un reste phényle, substitué éventuellement par halogène, phényle, phénoxy, alcoxy en C1-C4,
X représente C = 0 ou CHOH,
Y représente hydrogène, alkyle ayant 1 à 9 atomes C, alkyl-C1-C4-oxy, halogène, phényle, phénoxy,
n = 1 à 5,
ainsi que leurs sels et leurs composés à complexes métalliques.

2. Procédé de préparation de composés de formule générale I, caractérisé par le fait que l'on met à réagir des composés de formule générale II, 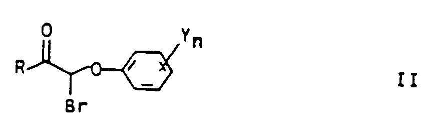 dans laquelle
R, Y et n ont les significations données dans la revendication 1, avec du 1-hydroxy-1,2,4-triazole et on réduit éventuellement les composés obtenus, de formule générale I (X = C = 0) en composés de formule générale I (X = CHOH).

3. Agents fongicides, caractérisés par une teneur en support et une quantité efficace au point de vue fongicide d'un composé de formule générale I, selon la revendication 1.

4. Procédé de lutte contre les champignons, caractérisé par le fait que l'on traite les champignons ou les plantes, semences, matériaux ou surfaces menacés par l'attaque par les champignons avec une quantité efficace au point de vue fongicide d'un composé de formule générale I, selon la revendication 1.

5. Composés de formule générale I, selon la revendication 1, dans laquelle
R = ter-butyle,
X = C=0 et
Yₙ = 4-chlore ou 2,4-dichlore.

6. Composés de formule générale I, selon la revendication 1, dans laquelle
R = ter-butyle,
X = CHOH et
Yₙ = 4-chlore ou 2,4-dichlore.
